(19) Europäisches Patentamt / European Patent Office / Office européen des brevets

(11) **EP 1 973 418 B1**

(12) **EUROPEAN PATENT SPECIFICATION**

(45) Date of publication and mention
of the grant of the patent:
**18.05.2016 Bulletin 2016/20**

(21) Application number: **06841186.7**

(22) Date of filing: **28.12.2006**

(51) Int Cl.:
*A23K 20/00* (2016.01)          *A23K 50/00* (2016.01)
*A61K 31/19* (2006.01)

(86) International application number:
**PCT/EP2006/012561**

(87) International publication number:
**WO 2007/079970 (19.07.2007 Gazette 2007/29)**

(54) **USE OF A NUTRACEUTICAL COMPOSITION IN ANIMAL FEED**

ANWENDUNG EINER NUTRAZEUTISCHEN ZUSAMMENSETZUNG IN TIERFUTTER

UTILISATION D'UNE COMPOSITION NUTRACEUTIQUE DANS L'ALIMENTATION ANIMALE

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.01.2006 EP 06000669**

(43) Date of publication of application:
**01.10.2008 Bulletin 2008/40**

(73) Proprietor: **DSM IP Assets B.V.**
**6411 TE Heerlen (NL)**

(72) Inventors:
• **GUGEENBUHL, Patrick**
**68127 Sainte Croix en Plaine (FR)**
• **SIMOES-NUNES, Carlos**
**68128 Village-neuf (FR)**

(74) Representative: **Schwander, Kuno et al**
**DSM Nutritional Products Ltd**
**Wurmisweg 576**
**4303 Kaiseraugst (CH)**

(56) References cited:
EP-A1- 0 683 985          WO-A-96/24248
WO-A-98/08499          GB-A- 2 008 381
US-B1- 6 322 807

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a novel use of a nutraceutical composition for animals, especially pigs, comprising as active ingredient benzoic acid, derivatives or metabolites thereof.

Specific examples of derivatives of benzoic acid which can give rise to benzoic acid in vivo include salts of benzoic acid such as the alkali metal-alkaline earth metal- and ammonium benzoates.

The term "nutraceutical" as used herein denotes a usefulness in both the nutritional and pharmaceutical field of application. Thus, the nutraceutical compositions can find use as a complete animal feed (diet), as supplement to animal feed, and as pharmaceutical formulations for enteral or parenteral application which may be solid formulations, or liquid formulations.

It is known from EP-A-0 683 985 that animal feed compositions comprising benzoic acid or salts thereof can be used to minimize the emission of odoriferous ammonia from organic wastes, especially animal excrements and manure.

It is also known from WO98/08499 that feed compositions comprising benzoic acid and protein sources inter alia 6.5% soya meal and 7.1% maize gluten feed can be used as a medicament for animals for promoting growth thereof and improving the digestibility of amino acids fed to such animals.

It has now been found surprisingly that in addition to the above function, benzoic acid and salts thereof have the advantage of being able to improve digestibility of proteins in animal feeds, i.e. to promote amino acid and nitrogen assimilation, and to increase total energy levels.

Moreover, it has been found that benzoic acid improves the digestibility of animal feed/feedstuff containing maize-soybean meal as a major part of the protein source.

The term "energy" and "energy level" as used herein denotes the total energy levels of the diets and ileal contents determined by calorimeteric measurement.

The term "nitrogen assimilation" and "total nitrogen" as used herein denotes the total nitrogen concentration determined on the freeze-dried diets and ileal contents.

The term "amino acid assimilation" and "total amino acids" as used herein denotes the total amino acids determined after an acidic hydrolysis on the freeze-dried diets and intestinal contents.

Accordingly, the present invention relates to a composition comprising benzoic acid or a derivative of benzoic acid capable of giving rise to free benzoic acid in vivo for improving the digestibility of proteins and/or the assimilation of amino acids and/or nitrogen in animals and/or to increase total energy levels in animal feedstuff.

Furthermore, the present invention relates to the novel use of benzoic acid in order to improve the digestibility of proteins and/or the assimilation of amino acids and/or nitrogen in animals and/or to increase total energy levels in animal feedstuff.

Particularly, the present invention relates to the novel use of benzoic acid or a derivative of benzoic acid in a feed composition comprising maize-soybean meal as a major part of protein source for the assimilation of amino acids and/or nitrogen in animals, and to increase total energy levels in animal feedstuff.

The animal may be selected from poultry, pigs and cattle.

Benzoic acid or a derivative thereof capable of giving rise to benzoic acid in vivo may be administrated to the animals as a component of a nutraceutical composition which is conventionally fed to animals. Thus, benzoic acid and derivatives thereof may be suitably administered to the animals as a component of the animal feed or in their drinking water.

The amount of benzoic acids or a derivative thereof administered to the animal is in the range from 0.001 - 5% based on the total weight of each feed fed to the animal. This amount may, however, be higher if the function of benzoic acid or a derivative thereof is also to control the pH of the animal excreta fed on such a diet in order to suppress the emission of ammonia from the excreta. Such higher amounts are suitably limited to a maximum of about 10% based on the total animal feed composition.

In a preferred embodiment of the invention benzoic acid or a derivative of benzoic acid being used in an amount sufficient to provide a daily dosage of 200 mg per kg body weight to about 600 mg per kg body weight of the subject to which it is to be administered.

Furthermore, the present invention relates to a method of feeding an animal which comprises providing to the animal a feedstuff comprising a protein source, wherein the assimilation of amino acid by the animal is above 80% w/w compared with the total amount of added amino acids and/or the assimilation of nitrogen by the animal is above 74% w/w compared with the total amount of added nitrogen.

Finally; the invention also relates to a method for increasing the amount of metabolizable nitrogen and/or total amino acids by an animal. This method comprises providing to the animal for ingestion thereof an effective amount of benzoic acid or a derivative of benzoic acid which is present as a ingredient of the feedstuff ingested by the animal.

A typical formulation for an animal feed composition is shown in Table 1 below in which all the amounts shown in % by weight were fed to pigs:

Table 1

| Ingredients | % |
|---|---|
| Meat meal (58% Crude protein) | 3.20 |
| Molasses | 5.00 |
| Wheat | 5.90 |
| Soybean meal (45% crude protein) | 15.10 |
| Tapioca (66% starch) | 35.50 |
| Wheat middlings | 15.00 |
| Animal fat | 3.30 |
| Limestone | 0.74 |
| Lysine hydrochloride (98%) | 0.06 |
| Vitamin premix | 0.50 |
| Trace minerals | 0.50 |
| Sunflower meal | 12.20 |
| Amonium chloride | 2.00 |
| Amonium benzoate | 1.00 |

Thus benzoic acid or a derivative thereof may be used in combination with conventional ingredients present in an animal feed composition (diet) such as calcium carbonates, elelctrolytes such as ammonium chloride, proteins such as soya bean meal, wheat, starch, sunflower meal, corn, meat and bone meal, amino acids, animal fat, vitamins and trace minerals.

In such a composition, the ratio of the electrolyte to the benzoic acid or a derivative thereof is suitably in the range from 0.5 : 1 to 5 : 1 w/w, preferably from 1.5 : 1 to 3 . 1 w/w.

Benzoic acid or a derivative thereof is particularly effective to improve the digestibility of proteins and the assimilation of amino acids and nitrogen in animals such as poultry, pigs or cattle, especially pigs.

The present invention is further illustrated with reference to the following Example, which shows the effects of benzoic acid on the ileal apparent digestibility of nitrogen, energy and amino acids in the ileo-rectal anastomosed piglets

*Animas trial*

Ten Large White x Landrace x Piétrain piglets were used. The animals were obtained from the GAEC Leclerc, F-68150 Ostheim and were 60 days old at the beginning of the experiment.

Each animal was subjected to an ileo-rectal anastomosis according to the techniques described by Laplace et al. (1994). After surgery piglets were housed individually in stainless-steel metabolic cages in an animal room under controlled temperature (22°C), humidity (50 %), ventilation (1100 m3/h) and lighting (12 hours light/12 hours darkness cycle). Animals had a 10 days post-surgical recovery period, also required to stabilize feed intake. Meanwhile they were fed ad libitum a vegetal based diet for piglets and had free access to tap water.

During the experimental period, piglets were fed each and alternatively the vegetal based diet supplemented or not with 0.5 % benzoic acid.

The feed composition and the 2 experimental diets are presented in tables 2 and 3 respectively.

Table 2

| Ingredients | (%) |
|---|---|
| Maize | 53.0 |
| Soybean meal | 18.0 |
| Barley | 13.0 |
| Oat meal | 6.0 |
| Wheat bran | 5.4 |

(continued)

| Ingredients | (%) |
|---|---|
| Soya oil | 1.0 |
| Minerals, vitamins and synthetic amino acids | 3.6 |
| Crude protein - N x 6.25 | 15.5 |
| Lysine | 0.96 |
| Methionine + Cystine | 0.54 |
| Estimated digestible energy | 13.31 MJ/kg |

An indigestible tracer, chromium oxide, was added at a concentration of 0.4 % to all the diets in order to measure ileal apparent digestibility.

Table 3

| Diet | A | B |
|---|---|---|
| 0.5 % benzoic acid supplemented | - | + |
| Programmed dosage (mg/kg) | 0 | 5 |

The experience was composed of 2 periods of 12 days each divided in 10 adaptation days and in 2 ileal content sampling days.

The design protocol is presented in table 4. Each animal was fed alternatively with each diet, so that each diet was tested 10 times.

The animal health status was controlled daily.

Table 4

| Experimental period | Animal | | | | | | | | | |
|---|---|---|---|---|---|---|---|---|---|---|
| | 1 | 2 | 3 | 4 | 5 | 6 | 7 | 8 | 9 | 10 |
| | Experimental diet | | | | | | | | | |
| Period 1 | A | B | A | B | A | B | A | B | A | B |
| Period 2 | B | A | B | A | B | A | B | A | B | A |

For the digestibility estimations, samples of ileal content were collected daily, during the 2 sampling days, from 8 AM to 3 PM, as soon as they were produced. Immediately after each sampling, the collected contents were weighed and frozen at -80°C. All the daily samples from the same piglet were bulked. Before analysis the samples were freeze dried and grounded to a fine powder. A total of 20 samples were obtained for each diet.

*Analytical methods*

The dry matter contents of the freeze-dried samples were determined after drying overnight at 105°C, 20 g of the diets and 15 g of the ileal samples, according to the standard Association of Official Analytical Chemists (AOAC) procedure (1990).

The diets and ileal samples chromium oxide levels were determined by inductively coupled plasma-atomic emission spectrometry (ICP) after mineralization at 350°C during 20 min with oxidant reagent.

The benzoic acid content in feed was determined by HPLC.

Total energy levels were determined on the freeze-dried diets and ileal contents using a calorimeter IKA C7000. Freeze-dried powder amounts of approximately 400 mg were analyzed 3 times for each sample.

Total nitrogen concentrations were determined on the freeze-dried diets and ileal contents using a nitrogen analyzer LECO FP-428.

Freeze-dried powder amounts of approximately 100 mg were analyzed 3 times for each sample.

Total amino acids were determined after an acidic hydrolysis on the freeze-dried diets and intestinal contents. Tryptophan analysis was omitted. Cys and Met were analyzed as cysteic acid and methionine sulfone after oxidation with formic acid and hydrogen peroxide followed by an acidic hydrolysis at 110°C with 6 N HCl All the other amino acids were analyzed after an acidic hydrolysis at 110°C with 6 N HCl.

Individual amino acid concentrations were measured after derivation using Waters AccQ.Fluor reagent and quantified by reversed-phase HPLC by fluorescence detection after elution on a Pico.Tag C18 column.

Data were recorded with the Waters Millenium software for data acquisition, integration and handling. Quantification was realized with an internal standard using peak area. The detail method was described in Roche R&D Memorandum B-171'680.

Freeze-dried powder amounts of approximately 50 mg were analyzed twice for each sample.

*Data Analysis*

Correcting with the dry matter, the chromium oxide and the diet tested parameter concentrations, all the tested parameter levels of the freeze-dried ileal contents were expressed in % apparent digestibility using the following formula:

$$Digestibility\ (\%) = 100 - \left[ \frac{(Ci \times Md)}{(Mi \times Cd)} \times 100 \right]$$

Ci = parameter concentration (% dry matter) in the ileal content
Cd = parameter concentration (% dry matter) in the diet
Mi = chromium oxide concentration (% dry matter) in the ileal content
Md = chromium oxide concentration (% dry matter) in the diet

For each test group, statistic analysis of the data involved the calculation of the mean and standard deviation of the mean as well as an analysis of variance followed by Student's "t" test to assess the significance of group differences between group A and benzoic acid supplemented group B for a probability of 10 % for the amino acids and 5 % for nitrogen and energy.

*Results*

At the beginning of the study all the piglets had recovered from the anesthesia and the surgery. None of the animals showed during the experimental period, any symptoms of illness or toxicosis. All the feedings and all the sampling could be achieved.

The results of the benzoic acid in feed contents are presented in table 5. Each diet was analyzed twice.

Table 5

| Diet | A | B |
|---|---|---|
| 0.5 % Benzoic acid supplemented | - | + |
| Programmed dosage (mg/kg) | 0.0 | 5.0 |
| Measured benzoic acid (mg/kg) | 0.0 | 4.6 |
| Recovery (%) | - | 92.0 |

The observed benzoic acid content in the supplemented feed was in agreement with the programmed inclusion level for diet B.

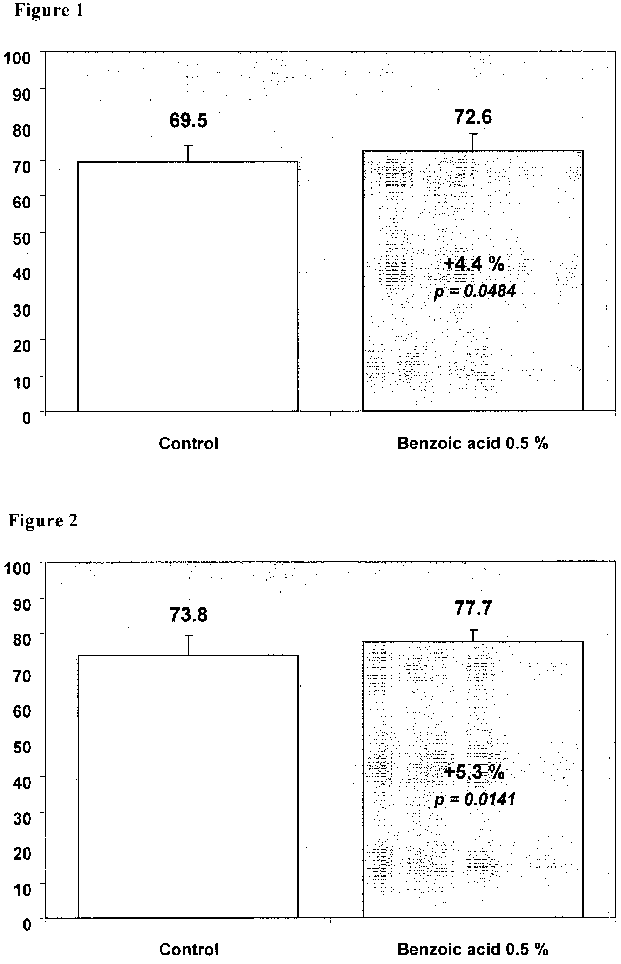

The results of the ileal apparent digestibility of energy are showed in figure 1. Figure 1 shows the ileal apparent energy digestibility in piglets fed a diet without or with supplementation of 0.5% benzoic acid. In the control non-supplemented diet, the ileal apparent energy digestibility was 69.5 ± 4.6 %. Compared to the control group, the ileal apparent energy digestibility of the 0.5 % benzoic acid supplemented group was 72.6 ± 4.7 %, representing a statistically significant ($p \leq 0.05$) increase of 4.4 %.

The results of the ileal apparent digestibility of total nitrogen are showed in figure 2. In the control non-supplemented diet, the ileal apparent total nitrogen digestibility was 73.8 ± 5.8 %. Compared to the control group, the ileal apparent total nitrogen digestibility of the 0.5 % benzoic acid supplemented group was 77.7 ± 3.1 %, representing a statistically significant ($p \leq 0.05$) increase of 5.3 %.

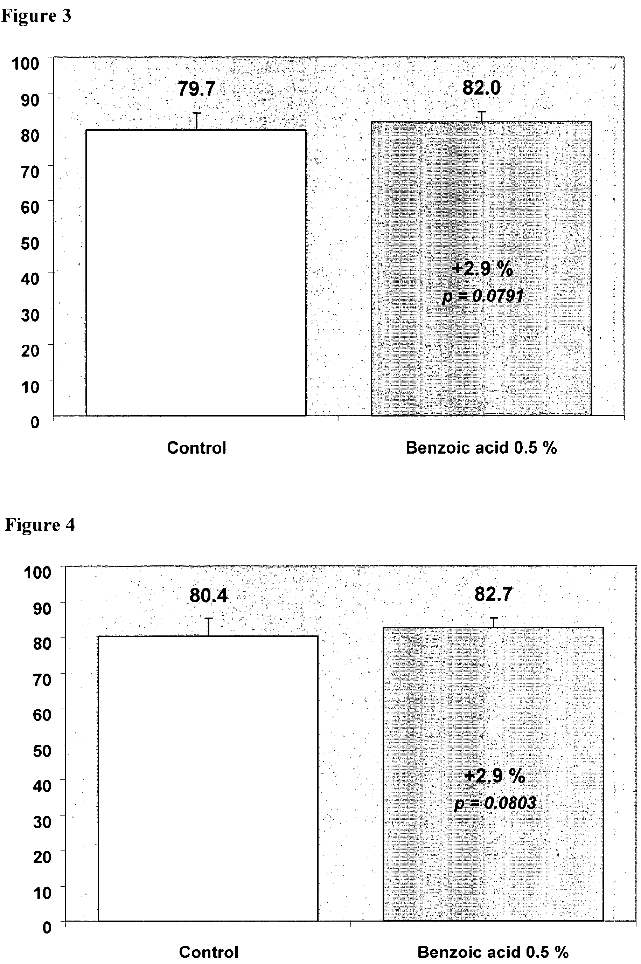

The results of the ileal apparent digestibility of total amino acids are showed in figure 3. In the control non-supplemented diet, the ileal apparent total amino acids digestibility was 79.7 ± 4.8 %. Compared to the control group, the ileal apparent total amino acids digestibility of the 0.5 % benzoic acid supplemented group was 82.0 $\pm$ 2.7 %, representing a statistically significant (p $\leq$ 0.10) increase of 2.9 %.

The essential amino acids can not be synthesized in sufficient amounts by the body and therefore must be supplied from the feed protein degradation. The essential amino acids in pigs are Arg, Cys[2], His, Ile, Leu, Lys, Met, Phe, Thr, Tyr, Val.

The results of the ileal apparent digestibility of essential amino acids are showed in figure 4. In the control non-supplemented diet, the ileal apparent essential amino acids digestibility was 80.4 $\pm$ 4.9 %. Compared to the control group, the ileal apparent essential amino acids digestibility of the 0.5 % benzoic acid supplemented group was 82.7 $\pm$ 2.6 %, representing a statistically significant (p $\leq$ 0.10) increase of 2.9 %.

There are 3 sulfured amino acids methionine (Met), cysteine (Cys) and cystine (Cys[2]). Under physiologic conditions Cys and Cys[2] are naturally inter-converted.

Besides the fact that Met and Cys are indispensable amino acids for the pig, they have also an essential role as constituent of all proteins.

Furthermore, Met plays also an important part in the protein synthesis and as a methyl group provider, and Cys takes part in the storage and transport of sulfur and in various synthesis like that of glutathione. Cys[2] is also necessary for the formation of skin.

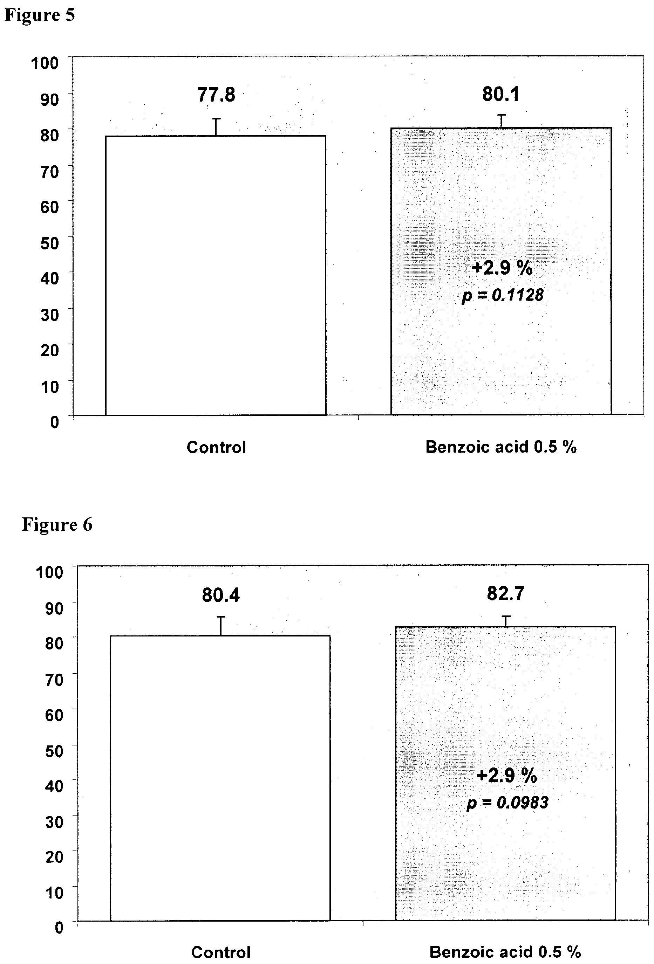

The results of the ileal apparent digestibility of sulfured amino acids are showed in figure 5. In the control non-supplemented diet, the ileal apparent sulfured amino acids digestibility was 77.8 $\pm$ 4.9 %. Compared to the control group, the ileal apparent sulfured amino acids digestibility of the 0.5 % benzoic acid supplemented group was 80.1 $\pm$ 3.5 %, representing a statistically non-significant (p $\leq$ 0.10) increase of 2.9 %.

Branched chain amino acids (Leu, Ile and Val) are needed for the maintenance of muscle tissue and to preserve glycogen stores as an energy source for muscle. They also help to prevent protein degradation during exercise and thereby to lead to a greater lean mass.

The results of the ileal apparent digestibility of branched chain amino acids are showed in figure 6. In the control non-supplemented diet, the ileal apparent branched chain amino acids digestibility was 80.4 $\pm$ 5.3 %. Compared to the control group, the ileal apparent branched chain amino acids digestibility of the 0.5 % benzoic acid supplemented group was 82.7 $\pm$ 2.8 %, representing a statistically significant (p $\leq$ 0.10) increase of 2.9 %.

Lysine is an essential amino acid in the strict sense that it is not biosynthesized in vivo at all. Lysine has a marked effect on growth. The low lysine content in vegetable proteins represents a significant problem for nutrition.

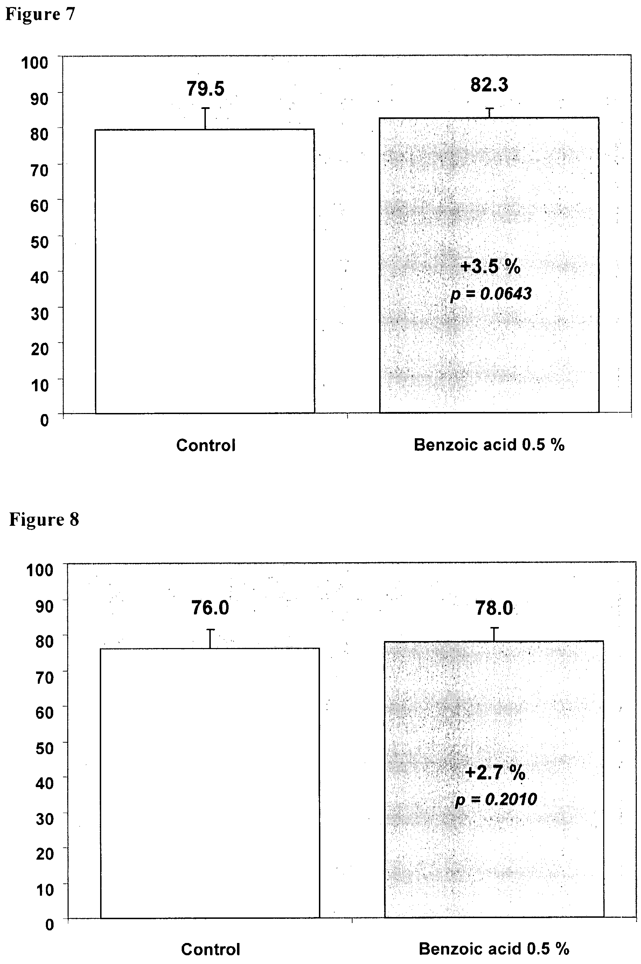

The results of the ileal apparent digestibility of lysine are showed in figure 7. In the control non-supplemented diet, the ileal apparent lysine digestibility was 79.5 $\pm$ 5.7 %. Compared to the control group, the ileal apparent lysine digestibility of the 0.5 % benzoic acid supplemented group was 82.3 $\pm$ 2.8 %, representing a statistically significant (p $\leq$ 0.10) increase of 3.5 %.

Threonine is like lysine an essential amino acid nutritionally indispensable for growth. The efficiency of threonine utilization by animal, from protein sources, is difficult due to the difficulty to break the peptidic linkages where threonine is involved. For this reason, threonine is considered to be the second required amino acid after lysine for supplementation in the diet.

The results of the ileal apparent digestibility of threonine are showed in figure 8. In the control non-supplemented diet, the ileal apparent threonine digestibility was 76.0 $\pm$ 5.5 %. Compared to the control group, the ileal apparent threonine digestibility of the 0.5 % benzoic acid supplemented group was 78.0 $\pm$ 3.9 %, representing a statistically non-significant (p $\leq$ 0.10) increase of 2.7 %.

Amino acids are first of all the stones of the protein synthesis and therefore the base of the muscle accretion. Most of them are glycogenic, some are also involved in the fatty acid metabolism. Amino acids are implicated in ammonia detoxification, are precursors of glutathion, creatine, hormones, neuropeptides, vitamins and other physiologic proteic compounds.

The results of the ileal apparent digestibility of the individual amino acids are showed in table 6.

Table 6

| Amino Acid | Mean digestibility $\pm$ $\sigma$ (%) | |
| --- | --- | --- |
| | Control | Benzoic acid 0.5 % |
| Ala | 72.1 $\pm$ 6.5 | 75.3 $\pm$ 3.7 |
| Arg | 84.8 $\pm$ 3.9 | 87.0 $\pm$ 1.8 |
| Asp+Asn | 76.1 $\pm$ 6.5 | 79.1 $\pm$ 3.2 |
| Cys | 77.2 $\pm$ 5.0 | 79.7 $\pm$ 4.4 |
| Glu+Gln | 84.8 $\pm$ 4.3 | 86.7 $\pm$ 2.1 |

(continued)

| Amino Acid | Mean digestibility ± σ (%) | |
| --- | --- | --- |
| | Control | Benzoic acid 0.5 % |
| Gly | 72.7 ± 5.3 | 75.1 ± 4.4 |
| His | 84.3 ± 4.6 | 86.4 ± 2.5 |
| Hyp | 39.8 ± 11.0 | 41.4 ± 13.4 |
| Ile | 78.5 ± 6.9 | 81.5 ± 3.6 |
| Leu | 82.8 ± 4.5 | 84.5 ± 2.4 |
| Lys | 79.5 ± 5.7 | 82.3 ± 2.8 |
| Met | 78.9 ± 5.4 | 80.7 ± 3.5 |
| Phe | 82.4 ± 4.6 | 84.5 ± 2.3 |
| Pro | 84.6 ± 3.1 | 85.8 ± 2.4 |
| Ser | 80.6 ± 4.5 | 82.6 ± 2.9 |
| Thr | 76.0 ± 5.5 | 78.0 ± 3.9 |
| Tyr | 78.6 ± 5.0 | 80.8 ± 2.8 |
| Val | 76.1 ± 6.4 | 79.5 ± 3.6 |

**Claims**

1. Use of benzoic acid or a derivative of benzoic acid in a feed composition comprising maize-soybean meal as a major part of protein source for the assimilation of amino acids and/or nitrogen in animals.

2. Use of benzoic acid or a derivative of benzoic in a feed composition comprising maize-soybean meal as a major part of protein source to increase total energy levels in animal feedstuff.

3. Use as in claim 1 or 2, wherein the animal is selected from poultry, pigs and cattle.

4. Use according to one of the claims 1 to 3, wherein said benzoic acid or a derivative of benzoic acid being used in an amount sufficient to provide a daily dosage of 200 mg per kg body weight to about 600 mg per kg body weight of the subject to which it is to be administered.

**Patentansprüche**

1. Verwendung von Benzoesäure oder einem Derivat der Benzoesäure in einer Futterzusammensetzung, die Mais-Sojabohnen-Mehl als Hauptbestandteil der Proteinquelle umfasst, für die Assimilation von Aminosäuren und/oder Stickstoff in Tieren.

2. Verwendung von Benzoesäure oder einem Derivat der Benzoesäure in einer Futterzusammensetzung, die Mais-Sojabohnen-Mehl als Hauptbestandteil der Proteinquelle umfasst, zum Erhöhen der Gesamtenergiegehalte in Tierfuttermitteln.

3. Verwendung nach Anspruch 1 oder 2, wobei das Tier aus Geflügel, Schweinen und Rindern ausgewählt ist.

4. Verwendung nach einem der Ansprüche 1 bis 3, wobei die Benzoesäure oder ein Derivat der Benzoesäure in einer Menge verwendet wird, die ausreicht, um eine Tagesdosis von 200 mg pro kg Körpergewicht bis ungefähr 600 mg pro kg Körpergewicht des Individuums, an das sie verabreicht werden soll, bereitzustellen.

**Revendications**

1. Utilisation d'acide benzoïque ou d'un dérivé d'acide benzoïque dans une composition d'alimentation comprenant un tourteau de maïs-soja en tant que composant majeur de source de protéine pour l'assimilation d'acides aminés et/ou d'azote chez des animaux.

2. Utilisation d'acide benzoïque ou d'un dérivé d'acide benzoïque dans une composition d'alimentation comprenant un tourteau de maïs-soja en tant que composant majeur de source de protéine pour augmenter les taux d'énergie totaux dans un aliment pour animaux.

3. Utilisation selon la revendication 1 ou 2, dans laquelle l'animal est choisi parmi des volailles, des porcs et des bovins.

4. Utilisation selon une des revendications 1 à 3, dans laquelle ledit acide benzoïque ou dérivé d'acide benzoïque est utilisé en une quantité suffisante pour obtenir une dose journalière de 200 mg par kg de poids corporel à environ 600 mg par kg de poids corporel du sujet auquel il doit être administré.

## Figure 1

## Figure 2

**Figure 3**

**Figure 4**

Figure 5

Figure 6

Figure 7

Figure 8

**EP 1 973 418 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0683985 A
- WO 9808499 A